# EUROPEAN PATENT APPLICATION

(11) **EP 4 491 120 A1**
(43) Date of publication of application: **15.01.2025**
(21) Application number: 23382725.2
(22) Date of filing: 14.07.2023
(51) Int. Cl.: A61B 5/279, A61B 5/391, A61B 5/00

(54) **ASSISTED REPRODUCTIVE TECHNIQUE CATHETER WITH UTERINE PERISTALSIS DETECTION**

(71) Applicant: Sonda Devices S.L., 46004 Valencia (ES)
(72) Inventor: ALBEROLA RUBIO, José, 46017 VALENCIA (ES); PELLICER MARTINEZ, Antonio, 46004 VALENCIA (ES)
(74) Representative: ZBM Patents - Zea, Barlocci & Markvardsen

(57) **Abstract**

There is presented an assisted reproductive technique catheter (1) comprising a lumen (15, 25, 35, 40) configured to receive an internal catheter for passage of an embryo-containing cannula or for direct passage of an embryo or embryos and configured to receive and allow the passage of a liquid substance, wherein the catheter comprises:
a first assembly (11, 21, 31) of electrodes, comprising:
- a first electrode (12, 22, 32, 42), the first electrode being a ring sensing electrode, concentrically arranged around the catheter; and
- a second electrode (13, 23, 33, 41), the second electrode being a ring sensing electrode, concentrically arranged around the catheter;
- wherein the first electrode and the second electrode are separated along a longitudinal axis (A) of the catheter by a first interelectrode separation (IE);
- and wherein the second electrode is arranged at a distal separation (DS) from a distal end (14, 24, 34) of the catheter. There is also provided a method for assisted reproductive technique.

## Description

### FIELD OF THE DISCLOSURE

The present disclosure relates to devices for assisted reproduction technology, in particular, the devices configured to measure uterine activity during a pre-transfer time period or during transfer of semen or an embryo containing solution into a uterus.

### BACKGROUND ART

Uterine peristalsis and myometrial activity are terms used to refer to contractions of the uterine muscle, which are associated with electrical activity of the muscle.

The uterine myometrium may be subject to variation in its contractile activity, which may vary from individual to individual and which may depend on factors such as the menstrual cycle, pathologies, and/or pregnancy.

An assisted reproduction technology, for example artificial insemination or in-vitro fertilization (IVF) and its derivatives, may comprise a placement of sperm or semen or embryos in a uterus of a female, possibly leading to an ongoing pregnancy. During placement of the embryos, an outer catheter may be inserted through the cervix into a uterine cavity. A further internal catheter may be inserted through the outer catheter, and its distal end may protrude from the outer catheter. The inner catheter is then positioned, to a point near the fundus, or top, of the uterine cavity. Finally, embryos and culture fluid for nourishment may be flushed through the inner catheter. All catheters may be finally withdrawn.

Monitoring of the uterus activity before sperm insertion or an embryo transfer or embryo transplantation may be performed using ultrasound imaging. There are, however, no currently known techniques or means for monitoring the uterine peristaltic activity with high degree of reliability before an embryo placement or embryo transfer occurs.

### SUMMARY

In an aspect of the disclosure, there is provided an assisted reproductive technique catheter comprising a lumen configured to receive an internal catheter for the passage of an embryo-containing cannula or direct passage of an embryo or embryos and configured to receive and allow the passage a liquid substance. Assisted reproductive technique comprises fertility treatments in which sperm or embryos are handled, for example, artificial Insemination (Al) or Intrauterine Insemination (IUI) which comprises depositing a semen sample, which has been prepared in advance in the laboratory, inside a woman's uterus in order to increase the potential of the spermatozoa and improve the chances of the egg being fertilized. Assisted reproductive technique comprises embryo transfer after an in-vitro fertilization, which comprises joining an egg with sperm in laboratory -in vitro- and then transferring an embryo resulting from the joining to a mother's uterus, possibly leading to a pregnancy. The assisted reproductive technique comprises a step in which a transfer of either semen or an embryo is performed. The catheter may be made of a biocompatible material, for example a biocompatible plastic.

The assisted reproductive technique catheter of the disclosure is configured to perform the transfer. For such transfer, a lumen is provided which is configured to receive an internal catheter for the passage of an embryo-containing cannula or direct passage of an embryo or embryos and configured to receive and allow the passage a liquid substance, for example semen. The lumen may comprise an inner diameter of the lumen equal or greater than 2.8 FR in the French scale, or equal or greater than 0,9 mm.

The electrodes may be made of an iridium palladium alloy. The outer diameter of the electrodes may be equal or greater than 6 FR in the French scale or equal or greater than 2 mm.

The distal separation DS may comprise a distance between 0 mm and 10mm, including 0 and 10 mm or the distal separation DS may be at least of 0,5 m, or 1 mm or 2mm, or 3mm, or the distal separation DS may be equal or less than 10 mm.

The catheter of the disclosure may be flexible. The catheter as described herein may be inserted in a vagina and displaced until the distal end of the catheter reaches the cervical canal and uterine cavity. The catheter may have a length of approximately between 20 and 50 cm. The lumen may present an inner diameter of the lumen equal or greater than 2.8 FR in the French scale, or equal or greater than 0,9 mm.

The catheter comprises a first assembly of electrodes, comprising a first electrode, the first electrode being a ring sensing electrode concentrically arranged around the catheter; and a second electrode, the second electrode being a ring sensing electrode concentrically arranged around the catheter. Uterine or myometrial peristalsis can be detected by processing the signal provided by the electrodes. Bipolar electrodes provide the advantage of providing located information about the myometrial walls. The fact of providing assemblies of two bipolar electrodes is that signal processing the signals received from both electrodes may provide information which is more precise in terms of information per mm2 area of a myometrial wall.

The electrodes are separated along a longitudinal axis of the catheter by a first interelectrode separation IE; and one of the electrodes, for example the second electrode, is arranged at a distal separation DS from a distal end of the catheter. In some examples the distal separation is substantially zero, whereas in other examples the distance separation is at least of 3 mm. The distal separation may be configurable by the user. The assembly of electrodes may be configured to be movable or displaceable. The catheter may comprise displacement means so that the catheter is configured to position the electrodes at a distal separation DS, chosen before or during use, from the distal end of the catheter. For example, the displacement means may comprise an inner helical machining of the electrodes, e.g. in the form of nuts, and the outer surface of the catheter, e.g. in the form of a screw, to displace the electrodes screwing them in a direction or an opposite direction depending on the separation to be obtained. For example, the displacement means may comprise an outer surface made of a material on which the electrodes may slide or glide. The displacement means may comprise a grove made in the outer surface of the catheter and a flap or flange in the electrodes, such that the flap may be displaced along the grove. The electrodes are configured to sense the myometrial activity when positioned inside a uterus. A practitioner may choose a distance IE or DS in which the electrodes need to be positioned depending on the anatomy of a woman's uterus or depending on the assisted reproductive technique to be performed. The displacement means may be configured to let the electrodes be displaced by application of a displacement force and pressure, for example applied by a user's fingers, and at the same time the displacement means may be configured to not let the electrodes be displaced or configured to maintain the electrodes in a desired position when the catheter is passed through the cervix. In other words, the displacement means may be configured to let the electrodes be displaced when a predetermined displacement pressure and/or force are applied to the electrodes. The predetermined displacement pressure and/or force are greater than the pressure and/or force the cervix applies to a catheter passing through. The IE and the DS as seen, may be configurable or may be fixed in manufacture. When configurable, and during use, a IE and a DS may be adjusted depending on the use or final assisted reproductive technique to be performed and, in examples, considering a minimum interelectrode separation, IE, of 1 mm.

Advantageously the assisted reproductive technique catheter of the disclosure is configured to be used for detecting peristalsis by measuring electrical properties of a uterus or myometrium right before or during or even after a transfer of a solution containing reproductive agents including any of semen, or sperm containing solution or embryo containing solution or solution to push an embryo attached to the distal end of the catheter, into a woman's uterus. The assisted reproductive technique catheter provides a reliable device to acquire signals which may be analyzed before or during the transfer of a solution containing reproductive agents.

ln a further aspect of the disclosure, there is provided a method for identifying a time of a likelihood over a predetermined threshold, or a highest likelihood, for assisted reproductive technique success in a subject, for example a woman, the method comprising positioning a catheter within a uterine cavity of the subject, the catheter being the assisted reproductive technique catheter of the disclosure, wherein positioning of the catheter comprises setting both electrodes in contact with at least a part of a myometrial wall and/or a myometrial cavity of the subject; receiving, from the first and second electrodes, electrical signals representative of the electro-physiological state of the uterine cavity, for example the myometrium; and identifying a time window with a likelihood over a predetermined threshold, or the highest likelihood, of assisted reproductive technique success. A predetermined threshold may be established by a practitioner and/or may be based on combination of parameters obtained or calculated base don't he electrical signals.

Positioning the catheter within a uterine cavity of the subject, comprises setting both electrodes in contact with at least a part of a myometrial wall and/or a myometrial cavity of the subject at a depth corresponding to any point along the myometrial wall necessary to analyze electrical signals from the electrodes. As the first electrode and second electrode of the catheter of the disclosure are known to be at an interelectrode separation, IE, plus a distal separation, DS, from the distal end of the catheter and at a distal separation DS from the distal end of the catheter respectively, a position in the uterus may be chosen so as to select the points from which one or more electrical signals is to be received.

Receiving, from at least the first and second electrodes, electrical signals representative of the electro-physiological state of the myometrium may comprise receiving a first electrical signal from a first electrode and receiving a second electrical signal from the second electrode. Receiving electrical signals may comprise receiving a first digital data and a second digital data. Receiving electrical signals may comprise receiving electrical signals during 2 minutes or during 3 minutes or during a larger time window of, for example, 10 minutes. The capture of electrical signals produced by muscles during a muscle contraction is known as electromyography. The electrical signals are generated by the exchange of ions across the membranes of muscle fibres due to muscle contraction. Electromyography (EMG) is the acquisition, recording and analysis of electrical activity generated in nerves and muscles through the use of electrodes, for example surface electrodes. The measurements extracted from EMG provide valuable information about physiology and muscle activation patterns. In the case of the disclosure, EMG is advantageously used by a catheter which is, in turn, used for the transfer of a solution containing reproductive agents.

ldentifying a time window with the highest likelihood of assisted reproductive technique success may be based on the electrical signals provided by the electrodes. Identifying a time window with the highest likelihood of assisted reproductive technique success may comprise identifying features appropriate for an assisted reproductive technique, such as an appropriate contraction frequency of the myometrial wall, a signal frequency average sensed by each of the electrodes. Some example features may comprise mean frequency, frequency median, standard deviation, ratio of high and low frequencies, first unnormalized moment, RMS, and entropy, or one or more of a contraction frequency, peak contraction intensity, mean contraction intensity, median contraction intensity, median contraction duration, mean contraction duration, peak contraction duration, shortest contraction duration, lowest contraction intensity, direction of propagation of a peristaltic wave, mean direction of propagation of a peristaltic wave, median direction of propagation of a peristaltic wave, peristaltic wave origin, mean peristaltic wave origin, median peristaltic wave origin, one or more features related to the differences between the peristaltic activity in different points of time, entropy, intensity/amplitude RMS, first normalized moment, contraction frequency standard deviation, ratio between high and low contraction frequencies, or any combination thereof, and/or any statistical representation of any order of the aforementioned features or any combination thereof. Contraction herein refers to uterine muscle contraction or peristaltic activity of the uterus or the myometrium. Any of the aforementioned features, and any combination thereof may be used to detect a contraction or a contraction event in the received electrical signals from the first and the second electrodes. For example, threshold values for each of the aforementioned features may be established, such that, a contraction or contraction event is positively detected based on a comparison between the measured or calculated value of the feature and the threshold value established for the feature. Thus, a discrete numerical and/or categorical characterization of the peristaltic activity may be obtained.

The advantages of a method according to the disclosure are varied and comprise the measurement, through the application of EMG techniques, of representative signals of the myometrium to assist and to identify an exact point of time when a transfer of a solution containing reproductive agents should be performed to obtain a highest probability of transfer success. A probability of transfer success may comprise a probability of pregnancy. Besides, the fact that the catheter of the disclosure need not be removed after the EMG and before the transfer, provides the further advantage that the cervix is not manipulated, or that the cervix remains right in the same conditions as when the electrical signals provided by the electrodes (regardless of the signal delay and signal processing delay, which may be considered insignificant or of little relevance for the technique) are analyzed and thus, the cervix remains in the same conditions as when identifying of a time window with the highest likelihood of assisted reproductive technique success.

Compared to prior art solutions where ultrasounds are performed to check the status of a uterus before a transfer and after some minutes or hours the transfer is performed, the catheter and method according to the present disclosure improves the current techniques.

### BRIEF DESCRIPTION OF THE DRAWINGS

Non-limiting examples of the present disclosure will be described in the following, with reference to the appended drawings, in which:
Figure 1 schematically illustrates an assisted reproductive technique catheter according to the disclosure.
Figure 2 schematically illustrates an assisted reproductive technique catheter according to the disclosure.
Figure 3 schematically illustrates an assisted reproductive technique catheter according to the disclosure.
Figure 4 schematically illustrates an assisted reproductive technique catheter according to the disclosure.
Figure 5 illustrates the example of figure 4 where a front view of the cut along line AB is shown.
Figure 6 schematically illustrates a system 6 comprising an assembly of electrodes and a bioamplifier.
Figure 7 schematically illustrates a system 6 comprising a catheter 7 comprising two assemblies 71 and 72 of electrodes and a bioamplifier.
Figure 8 represents an example of a method 8 according to the disclosure for identifying a time of highest likelihood for assisted reproductive technique success in a subject.

### DETAILED DESCRIPTION OF EXAMPLES

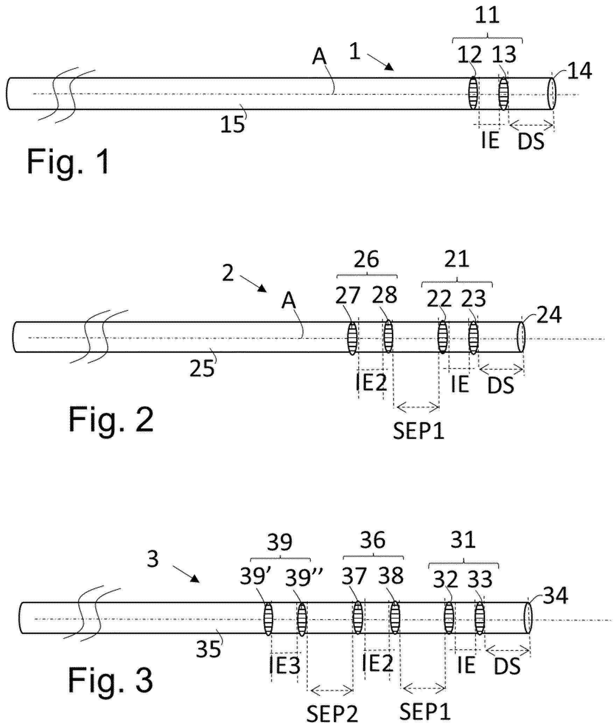

Figure 1 schematically illustrates an assisted reproductive technique catheter 1 comprising a lumen 15 configured to receive an internal catheter for the passage of an embryo-containing cannula or direct passage of an embryo or embryos and configured to receive and allow the passage a liquid substance, wherein the catheter 1 comprises a first assembly 11 of electrodes, comprising: a first electrode 12, the first electrode being a ring sensing electrode concentrically arranged around the catheter 10; and a second electrode 13, the second electrode being a ring sensing electrode concentrically arranged around the catheter 1; wherein the first electrode 12 and the second electrode 13 are separated along a longitudinal axis A of the catheter by a first interelectrode separation IE; and wherein the second electrode 13 is arranged at a distal separation DS from a distal end 14 of the catheter 1. The electrodes have a ring portion, which portions may be monolithically formed or separately integrated. The electrical activity sensed by each of the electrodes may be output via the catheter's wires as an analog signal. Thus, the catheter generates a set of analog signals during a measurement session. A catheter comprising two electrodes, as shown in Figure 1, may generate a set of two analog signals during a measurement session. The analog signals may be transmitted to a display means, such as a monitor or screen, for visualization, which may be referred to as an electromyogram. Analog signals may be transmitted to a processor. In order to transmit the analog signals generated by the electrodes to a processor and/or to visualization means, a connector, for example, a REDEL connector or other suitable connector, may be attached to the catheter. IE may be comprised in a range from 1 mm to 5 mm. DS may be comprised in a range of 0 mm to 10 mm.

Figure 2 schematically illustrates an assisted reproductive technique catheter 2 comprising the same features as the catheter of figure 1 and further comprising a second assembly 26 of electrodes, wherein the second assembly 26 of electrodes comprises at least: a third electrode 27, the third electrode being a ring sensing electrode concentrically arranged around the catheter 2; and a fourth electrode 28, the fourth electrode being a ring sensing electrode concentrically arranged around the catheter 2; wherein the third electrode 27 and the fourth electrode 28 are separated by a second interelectrode separation IE2; and wherein the second assembly 26 is arranged at a first separation SEP1 from the first assembly 21, wherein the first separation SEP1 is comprised in a range from 3 mm to 7 mm. IE may be different from IE2, and IE2 may be comprised in a range from 1 mm to 5 mm. The separation SEP1 comprises a distance from the fourth electrode 28, comprised in the second assembly, to the first electrode 22 comprised in the first assembly. The separation SEP1 may be configured to detect a contractile wave of interest. The separation between assemblies of electrodes allows sensing points in the uterine cavity which are separated by a controlled or configurable distance, SEP1, so that the obtained signal is representative of the muscular activity of exact points in the uterine cavity.

Figure 3 schematically illustrates an assisted reproductive technique catheter 3 comprising the same features as the catheter of figure 2 and further comprising a third assembly 39 of electrodes, wherein the third assembly 39 of electrodes comprises at least a fifth electrode 39', the fifth electrode being a ring sensing electrode concentrically arranged around the catheter 3; and a sixth electrode 39", the sixth electrode being a ring sensing electrode concentrically arranged around the catheter 3; wherein the fifth electrode 39' and the sixth electrode 39" are separated by a third interelectrode separation IE3; and wherein the third assembly 39 is arranged at a second separation SEP2 from the second assembly 36 of the catheter 3, wherein the second separation SEP2 is comprised in a range from 3 mm to 7 mm. The second separation SEP2 comprises a distance from the sixth electrode 39", comprised in the third assembly 39, to the third electrode 37 comprised in the second assembly 36.

SEP1 may be different from SEP2. IE3 may be comprised in a range from 1 mm to 5 mm. IE3 may be different from IE and from IE2.

ln examples, the first interelectrode separation (IE), and/or the second interelectrode separation (IE2) and/or the third interelectrode separation (IE3), is comprised in a range from 1 mm to 5 mm. The distances may be different from one another.

In examples, each one of the electrodes is connected by a respective electrical wire to a connector 48 at a proximal end 47 of the catheter. The electrical wires may be made of copper, of optical fiber, for example.

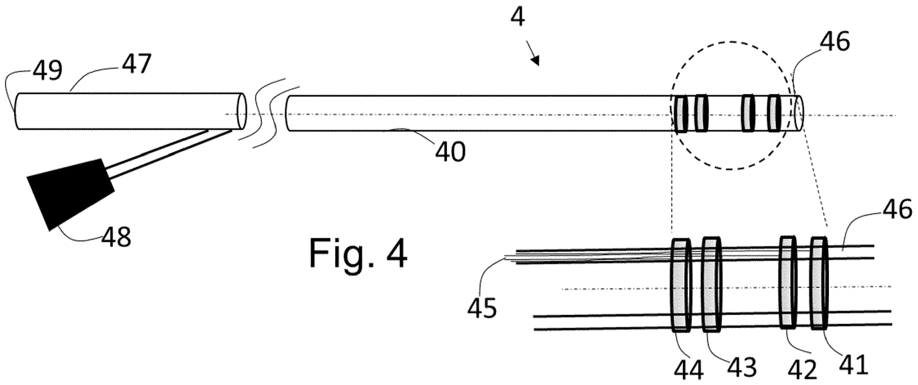

Figure 4 illustrates the catheter of figure 3 further comprising a coating 46; wherein each one of the electrodes 41, 42, 43, 44 are concentrically arranged around the catheter 4 and over the coating 46; and wherein each respective electrical wire 45 is arranged along the catheter 4 from a respective electrode to the connector 48 and is embedded in the coating 46 or arranged between the coating and the catheter. The connector 48 is provided so that the wires can leave the catheter and may connect to an external processing unit. The catheter comprises an entry 49 to insert a cannula or syringe to insert a solution containing reproductive agents into the uterus through the catheter. The entry 49 may comprise flange not shown for attaching an injection syringe or cylinder for the introduction of a solution into the lumen 40. Figure 4 shows a zoomed portion of the catheter 4 showing wires 45 running embedded inside the coating 46.

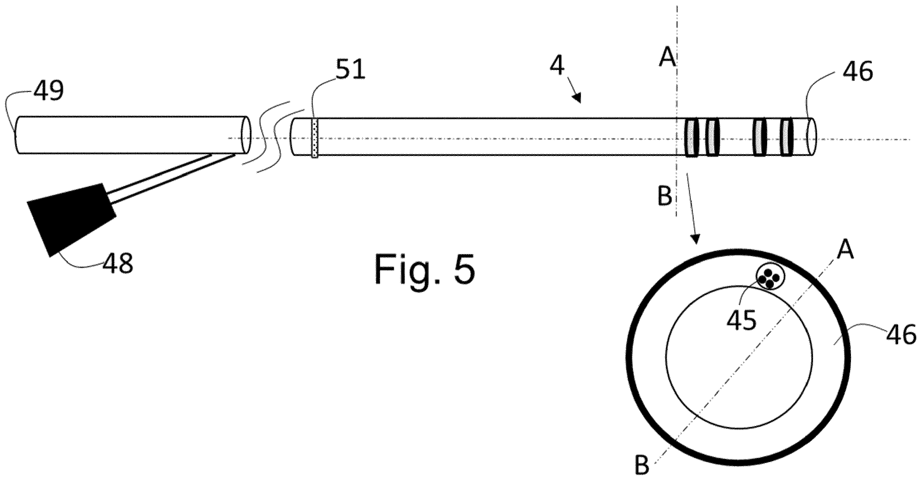

Figure 5 illustrates the example of figure 4 where a front view of the cut along line AB is shown. The wires (45) are shown inside a tubular arrangement in the coating (46).

ln examples, one or more of the wires may be comprised in between the coating and the catheter or embedded in the coating in a loose manner, or loosely embedded or embedded with certain tolerance to be displaced so that, when the electrodes are displaced for varying any of DS, SEP, SEP2, IE, IE2, the wires may provide a certain tolerance for movement. The length of a wire between an electrode and the connector (48) may vary and may allow the electrode to be moved in a range of 1mm to 3mm both included.

In examples where DS, SEP, SEP2, IE, IE2, are fixed or predetermined, and the catheter is not configured to move, the wires may be tightly embedded or arranged between the coating and the catheter or the wires may not be allowed to be moved. In those examples, the wires may be tightly embedded or arranged between the coating and the catheter or the wires may also be loosely embedded or embedded with certain tolerance to be displaced.

In some examples the coating is made of a biocompatible material or plastic. The coating may be flexible and the catheter may be flexible.

In examples, as shown in figure 5, the catheter comprises a stopper 51 at the entry of the cervix, configured to stop the catheter and prevent a user to insert the catheter forward during a vaginal insertion of the catheter. The stopper may be positioned at 4 cm or 5 cm from the distal end of the catheter. The stopper may be fixed or may be configured to be displaced from substantially 0,5cm to 1cm or 2cm or from 0,5 cm to 5 cm. The stopper 51 may be configured be displaced by application of a displacement force and pressure, for example applied by a user's fingers. The stopper may be displaceable by comprising, for example, an inner helical machining, e.g., in the form of a nut, and the outer surface of the catheter may present, e.g., the form of a screw along the length occupied by the stopper, to displace the stopper screwing the stopper in a direction or an opposite direction depending on the displacement to be obtained. The stopper 51 may be comprised in any of the examples shown in figures 1 to 7 in the disclosure.

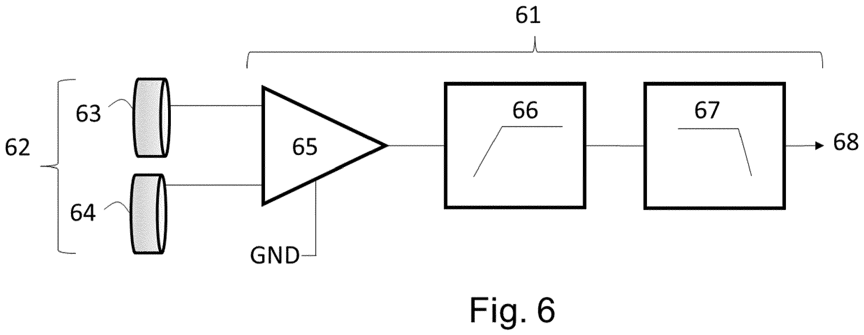

Figure 6 schematically illustrates a system 6 comprising an assembly of electrodes and a bioamplifier 61. For illustrative purposes, the electrodes are shown not arranged around the catheter of the disclosure. In some examples, as illustrated in figure 6, the bioamplifier 61 comprises at least one operational amplifier 65. The bioamplifier processes the two received signals from the electrodes 63 and 64. Figure 6 represents a schematic example of a first electrode 63 of the catheter in electrical connection with a first input pin of the operational amplifier 65 and a second electrode 64 in electrical connection with a second input pin of the operational amplifier 65. The first electrode and the second electrode belong to a same assembly of electrodes. In examples, as the one illustrated in figure 6, the system further comprises at least one or more of a high-pass filter 66 and/or a low-pass filter 67.

Bipolar electrodes provide the advantage of providing located information about the myometrial walls. Monopolar electrodes may provide more generic information of a muscle. The fact of providing assemblies of two bipolar electrodes is that signal processing the signals received from both electrodes may provide information which is more precise in terms of information per mm2 area of a myometrial wall. A bipolar signal is provided by calculating the difference between the voltage amplitude provided by one of the electrodes minus the voltage amplitude provided by the other electrode. The high-pass filter may comprise a cut-off frequency of 0.1 Hz. The low-pass filter may comprise a cut-off frequency of 25Hz.

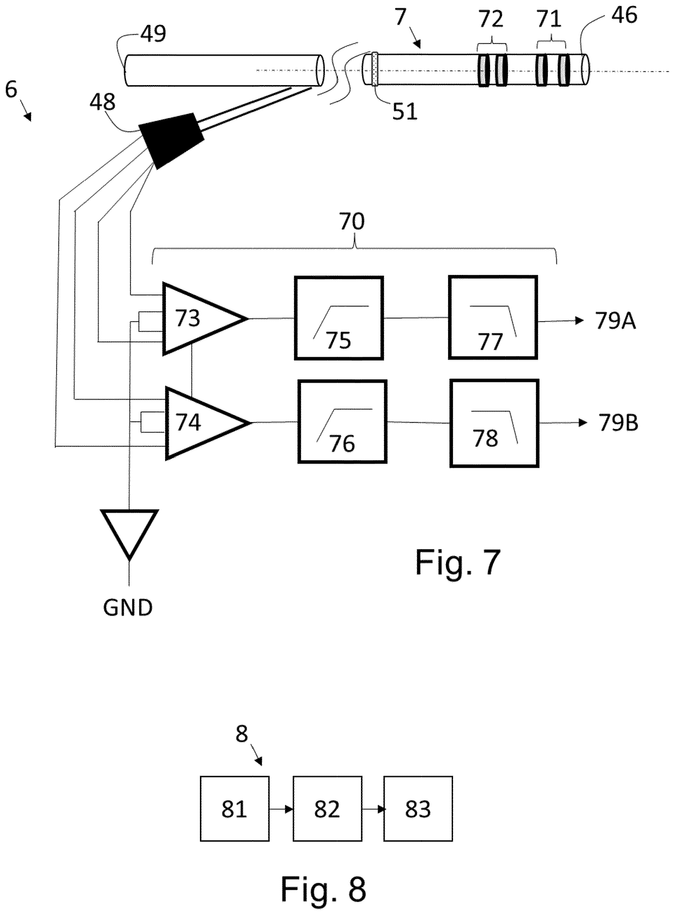

Figure 7 schematically illustrates a system 6 comprising a catheter 7 comprising two assemblies 71 and 72 of electrodes and a bioamplifier 70. The electrodes are shown arranged around a catheter 7 of the disclosure. The wires coming from the assembly 71 may, for example, feed the operational amplifier 73. The result of the difference provided by operational amplifier 73 between the voltage amplitude signals of the two electrodes of the assembly 71 may be provided to filter 75, which output may feed a further filter 77. The wires coming from the assembly 72 may, for example, feed the operational amplifier 74. The result of the difference between the voltage amplitude signals of the two electrodes of the assembly 72 may be provided to filter 76, which output may feed a further filter 78. The outputs 79A and 79B may be processed to interpret a contractile wave of the myometrial walls for example. Such a contractile wave is represented by the electrical signals provided by the first and second assemblies of electrodes at different heights or points of the uterine cavity. The contractile wave can therefore be represented in two different points of the uterine cavity at the same time. A similar arrangement may be provided for 3 assemblies, and a contractile wave can therefore be represented in three different points of the uterine cavity at the same time. As a contractile wave, theoretically, propagates in a uterine cavity from the fallopian tubes to the cervix, the contractile wave propagation may be followed by the arrangement of assemblies of electrodes in the catheter of the disclosure.

Figure 8 represents an example of a method 8 according to the disclosure for identifying a time of highest likelihood for assisted reproductive technique success in a subject, the method comprising, in block 81, positioning a catheter within a uterine cavity of the subject, wherein positioning comprises setting the first electrode and the second electrode in contact with at least part of a myometrial wall and/or a myometrial cavity of the subject. In some examples the distance of the first assembly of electrodes from the cervix may be configurable depending on the user or assisted reproductive technique needs. The method comprises, in block 82, receiving, from the first and second electrodes, electrical signals representative of the electro-physiological state of the myometrium; and the method comprises, in block 83, identifying a time window with the highest likelihood of assisted reproductive technique success a based at least in part in the electrical signals.

In some examples, a method for assisted reproductive technique may comprise performing the method identifying a time of highest likelihood for assisted reproductive technique success in a subject and may further comprise, after identifying the time window with the highest likelihood, and before removing the catheter from the uterine cavity, performing the assisted reproductive technique using the catheter during the time window with the highest likelihood. the method further comprises, after identifying the time window with the highest likelihood, and before removing the catheter from the uterine cavity, performing the assisted reproductive technique using the catheter during the time window with the highest likelihood. The time window with the highest likelihood may comprise several minutes, or may comprise several seconds, for example, between 1 minute to 5 minutes. The time window may comprise checking during approximately 2 minutes or 3 minutes that predetermined features are optimum and then, transfer a solution containing reproductive material or agents, for example sperm or an embryo. Some example features may comprise mean frequency, frequency median, standard deviation, ratio of high and low frequencies, first unnormalized moment, RMS, and entropy, or one or more of a contraction frequency, peak contraction intensity, mean contraction intensity, median contraction intensity, median contraction duration, mean contraction duration, peak contraction duration, shortest contraction duration, lowest contraction intensity, direction of propagation of a peristaltic wave, mean direction of propagation of a peristaltic wave, median direction of propagation of a peristaltic wave, peristaltic wave origin, mean peristaltic wave origin, median peristaltic wave origin, one or more features related to the differences between the peristaltic activity in different points of time, entropy, intensity/amplitude root mean square (RMS), first normalized moment, contraction frequency standard deviation, ratio between high and low contraction frequencies, or any combination thereof, and/or any statistical representation of any order of the aforementioned features or any combination thereof. Contraction herein refers to uterine muscle contraction or peristaltic activity of the uterus or the myometrium. Any of the aforementioned features, and any combination thereof may be used to detect a contraction or a contraction event in the received electrical signals from the first and the second electrodes. For example, threshold values for each of the aforementioned features may be established, such that, a contraction or contraction event is positively detected based on a comparison between the measured or calculated value of the feature and the threshold value established for the feature. Thus, a discrete numerical and/or categorical characterization of the peristaltic activity of the myometrium may be obtained.

The method may further comprise, in parallel or after block 82, and before block 83, receiving, from third and fourth electrodes, electrical signals representative of the electro-physiological state of the myometrium at a point where a second assembly comprising the third and fourth electrodes may be positioned. The method may further comprise, in parallel or after block 82, and before block 83, receiving, from fifth and sixth electrodes, electrical signals representative of the electro-physiological state of the myometrium at a point when a third assembly comprising the fifth and sixth electrodes may be positioned. The method may further comprise, in block 83, identifying a time window with the highest likelihood of assisted reproductive technique success based on the signals received by any one or more of third, fourth, fifth and sixth electrodes. These methods may be used to detect a contraction or a contraction event in the received electrical signals from any one, two or more of the first, second, third, fourth, fifth and sixth electrodes.

Although only a number of examples have been disclosed herein, other alternatives, modifications, uses and/or equivalents thereof are possible. Furthermore, all possible combinations of the described examples are also covered. Thus, the scope of the present disclosure should not be limited by particular examples but should be determined only by a fair reading of the claims that follow.

## Claims

1. An assisted reproductive technique catheter (1, 2, 3, 4, 7) comprising a lumen (15, 25, 35, 40) configured to receive an internal catheter for passage of an embryo-containing cannula or for direct passage of an embryo or embryos and configured to receive and allow the passage of a liquid substance, wherein the catheter comprises:
a first assembly (11, 21, 31) of electrodes, comprising:
- a first electrode (12, 22, 32, 42), the first electrode being a ring sensing electrode, concentrically arranged around the catheter; and
- a second electrode (13, 23, 33, 41), the second electrode being a ring sensing electrode, concentrically arranged around the catheter;
- wherein the first electrode and the second electrode are separated along a longitudinal axis (A) of the catheter by a first interelectrode separation (IE);
- and wherein the second electrode is arranged at a distal separation (DS) from a distal end (14, 24, 34) of the catheter.

2. The catheter (2, 3, 4, 7) of claim 1 further comprising a second assembly (26, 36) of electrodes, wherein the second assembly (26, 36) of electrodes comprises at least:
- a third electrode (27, 37, 44), the third electrode being a ring sensing electrode, concentrically arranged around the catheter; and
- and a fourth electrode (28, 38, 43), the fourth electrode being a ring sensing electrode concentrically arranged around the catheter;
- wherein the third electrode (27, 37, 44) and the fourth electrode (28, 38, 43) are separated by a second interelectrode separation (IE2);
- and wherein the second assembly (26, 36) is arranged at a first separation (SEP1) from the first assembly (21), wherein the first separation (SEP1) is comprised in a range from 3 mm to 7 mm.

3. The catheter (3) of claim 2 further comprising a third assembly (39) of electrodes, wherein the third assembly (39) of electrodes comprises at least:
- a fifth electrode (39'), the fifth electrode being a ring sensing electrode concentrically arranged around the catheter (3); and
- a sixth electrode (39"), the sixth electrode being a ring sensing electrode concentrically arranged around the catheter (3);
wherein the fifth electrode (39') and the sixth electrode (39") are separated by a third interelectrode separation (IE3);
- and wherein the third assembly (39) is arranged at a second separation (SEP2) from the second assembly (36) of the catheter (3), wherein the second separation (SEP2) is comprised in a range from 3 mm to 7 mm.

4. The catheter (1, 2, 3, 4, 7) of any one of claims 1 to 3, wherein the first interelectrode separation (IE), and/or the second interelectrode separation (IE2) and/or the third interelectrode separation (IE3), is comprised in a range from 1 mm to 5 mm.

5. The catheter (1, 2, 3, 4, 7) of any one of claims 1 to 4,
wherein each one of the electrodes is connected by a respective electrical wire to a connector (48) at a proximal end (47) of the catheter.

6. The catheter (1, 2, 3, 4, 7) of claim 5 further comprising a coating (46);
wherein each one of the electrodes (41, 42, 43, 44) are further arranged around the catheter (4) and over the coating (46); and
wherein each respective electrical wire (45) is arranged along the catheter (4) from a respective electrode to the connector (48) and is embedded in the coating (46) or arranged between the coating and the catheter.

7. The catheter (1, 2, 3, 4, 7) of claim 6 wherein the coating is made of a biocompatible material or plastic.

8. The catheter (1, 2, 3, 4, 7) of any one of claims 1 to 7 wherein an inner diameter of the lumen (15, 25, 35, 40) is equal or greater than 2.8 FR in the French scale, or equal or greater than 0,9 mm.

9. The catheter (1, 2, 3, 4, 7) of any one claims 1 to 8, wherein an outer diameter of the electrodes is equal or greater than 6 FR in the French scale or equal or greater than 2 mm.

10. The catheter (1, 2, 3, 4, 7) of any one of claims 1 to 9, wherein the distal separation (DS) is a distance between 0 and 10mm or wherein the distal separation (DS) is at least of 3mm.

11. The catheter of any one of claims 1 to 10 further comprising a stopper (51).

12. A system (6) comprising a catheter according to any one of claims 1 to 10 and a bioamplifier (61).

13. The system (6) of claim 12 wherein the bioamplifier (61) comprises at least one operational amplifier (65), wherein:
a first electrode (63) of the catheter (1, 2, 3, 4) is in electrical connection with a first input pin of the operational amplifier (65);
a second electrode (64) is in electrical connection with a second input pin of the operational amplifier (65);
wherein the first electrode and the second electrode belong to a same assembly (11, 21, 26, 31, 36, 39) of electrodes.

14. The system (6) of claim 12 or 13 further comprising at least one or more of a high-pass filter (66) and/or a low-pass filter (67).

15. A method for identifying a time a likelihood over a predetermined threshold for assisted reproductive technique success in a subject, the method comprising:
- positioning a catheter within a uterine cavity of the subject,
- the catheter comprising a lumen, the lumen being configured to receive an internal catheter for a passage of an embryo-containing cannula or direct passage of an embryo or embryos, wherein
- the catheter comprises at least a first assembly of electrodes comprising a first ring sensing electrode concentrically arranged around the catheter; and a second ring sensing electrode concentrically arranged around the catheter;
- wherein the first ring sensing electrode and the second ring sensing electrode are separated by a first interelectrode separation;
- and wherein the first assembly of electrodes is arranged at a distal separation from a distal end of the catheter;
- wherein positioning comprises setting at least the first electrode and the second electrode in contact with at least part of a myometrial wall and/or a myometrial cavity of the subject;
- receiving, from at least the first ring sensing electrode and second ring sensing electrode, electrical signals representative of an electro-physiological state of the uterine cavity; and
- identifying a time window with a likelihood over a predetermined threshold of assisted reproductive technique success based at least in part in the electrical signals.
